(19) Europäisches Patentamt / European Patent Office / Office européen des brevets

(11) **EP 2 511 878 B1**

(12) **EUROPEAN PATENT SPECIFICATION**

(45) Date of publication and mention
of the grant of the patent:
**06.05.2020  Bulletin 2020/19**

(51) Int Cl.:
***G06T 15/08*** *(2011.01)*

(21) Application number: **12163628.6**

(22) Date of filing: **10.04.2012**

(54) **Providing three-dimensional ultrasound image based on three-dimensional color reference table in ultrasound system**

Bereitstellung eines dreidimensionalen Ultraschallbildes basierend auf der dreidimensionalen Farbreferenztabelle in einem Ultraschallsystem

Fourniture d'une image à ultrasons tridimensionnelle basée sur une table de référence de couleurs tridimensionnelle dans un système à ultrasons

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **12.04.2011  KR 20110033913**

(43) Date of publication of application:
**17.10.2012  Bulletin 2012/42**

(73) Proprietor: **Samsung Medison Co., Ltd.**
**Kangwon-do 250-875 (KR)**

(72) Inventors:
 • **Na, Kyung Gun**
   **Seoul 135-851 (KR)**
 • **Kim, Sung Yun**
   **Seoul 135-851 (KR)**

(74) Representative: **Schmid, Wolfgang**
**Lorenz & Kollegen**
**Patentanwälte Partnerschaftsgesellschaft mbB**
**Alte Ulmer Strasse 2**
**89522 Heidenheim (DE)**

(56) References cited:
**US-A1- 2002 172 409**

 • **Alan Watt: "3D Computer Graphics, Third Edition", 2000, ADDISON WESLEY, London, XP002681439, * page 171 - page 183 * * page 370 - page 391 ***
 • **LEVOY M: "Display of surfaces from volume data", IEEE COMPUTER GRAPHICS AND APPLICATIONS, IEEE SERVICE CENTER, NEW YORK, NY, US, vol. 8, no. 3, 1 May 1988 (1988-05-01), pages 29-37, XP011413670, ISSN: 0272-1716, DOI: 10.1109/38.511**
 • **HÖNIGMANN D ET AL: "Adaptive design of a global opacity transfer function for direct volume rendering of ultrasound data", VIS 2003. IEEE VISUALIZATION 2003. PROCEEDINGS. SEATTLE, WA, OCT. 19 - 24, 2003; [ANNUAL IEEE CONFERENCE ON VISUALIZATION], NEW YORK, NY : IEEE, US, 1 January 2003 (2003-01-01), pages 489-496, XP031173536, DOI: 10.1109/VISUAL.2003.1250411 ISBN: 978-0-7803-8120-9**
 • **ZIV SOFERMAN ET AL: "Advanced Graphics Behind Medical Virtual Reality: Evolution of Algorithms, Hardware, and Software Interfaces", PROCEEDINGS OF THE IEEE, IEEE. NEW YORK, US, vol. 86, no. 3, 1 March 1998 (1998-03-01), XP011043998, ISSN: 0018-9219**
 • **ROBB R A: "Visualization in biomedical computing", PARALLEL COMPUTING, ELSEVIER PUBLISHERS, AMSTERDAM, NL, vol. 25, no. 13-14, 1 December 1999 (1999-12-01), pages 2067-2110, XP004363672, ISSN: 0167-8191, DOI: 10.1016/S0167-8191(99)00076-9**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present disclosure generally relates to ultrasound systems, and more particularly to providing a three-dimensional ultrasound image based on a three-dimensional color reference table in an ultrasound system.

BACKGROUND

An ultrasound system has become an important and popular diagnostic tool since it has a wide range of applications. Specifically, due to its non-invasive and non-destructive nature, the ultrasound system has been extensively used in the medical profession. Modern high-performance ultrasound systems and techniques are commonly used to produce two-dimensional or three-dimensional ultrasound images of internal features of target objects (e.g., human organs).

The ultrasound system provides a three-dimensional ultrasound image including clinical information, such as spatial information and anatomical figures of the target object, which cannot be provided by a two-dimensional ultrasound image. The ultrasound system transmits ultrasound signals to a living body including the target object and receives ultrasound echo signals reflected from the living body. The ultrasound system further forms volume data based on the ultrasound echo signals.

The ultrasound system further performs volume rendering upon the volume data to thereby form the three-dimensional ultrasound image.

When performing volume rendering upon the volume data based on ray-casting, it is required to calculate a gradient corresponding to each of the voxels of the volume data. Since a substantial amount of calculations and time are required to calculate the gradient corresponding to each of the voxels, the gradient is calculated at a preprocessing stage prior to performing volume rendering. However, a problem with this is that volume rendering (i.e., ray-casting) cannot be performed in a live mode for rendering the volume data acquired in real-time, based on the gradient.

An ultrasound system according to the preamble of claim 1 and a method of providing a three-dimensional ultrasound image according to the preamble of claim 7 are known from US 2002/172409A1. US 6559843 B1 discloses a method for rendering volumetric data having intensity values onto a viewing device using an array of processors. In particular, it discloses how to accumulate a color value of the pixel

SUMMARY

There are provided embodiments for providing a three-dimensional ultrasound image based on a three-dimensional color reference table for providing colors corresponding to at least one of intensity accumulation values and shading values throughout depth.

In one embodiment, by way of non-limiting example, an ultrasound system according to claim 1 is disclosed.

In another embodiment, there is provided a method of providing a three-dimensional ultrasound image according to claim 5.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

BRIEF DESCRIPTION OF THE DRAWINGS

FIG. 1 is a block diagram showing an illustrative embodiment of an ultrasound system.
FIG. 2 is a block diagram showing an illustrative embodiment of an ultrasound data acquisition unit.
FIG. 3 is a schematic diagram showing an example of acquiring ultrasound data corresponding to a plurality of frames.
FIG. 4 is a flow chart showing a process of forming a three-dimensional color reference table.
FIG. 5 is a schematic diagram showing an example of volume data.
FIG. 6 is a schematic diagram showing an example of a window.
FIG. 7 is a schematic diagram showing an example of polygons and surface normals.
FIG. 8 is a flow chart showing a process of forming a three-dimensional ultrasound image,

DETAILED DESCRIPTION

A detailed description is provided with reference to the accompanying drawings. One of ordinary skill in the art should recognize that the following description is illustrative only and is not in any way limiting.

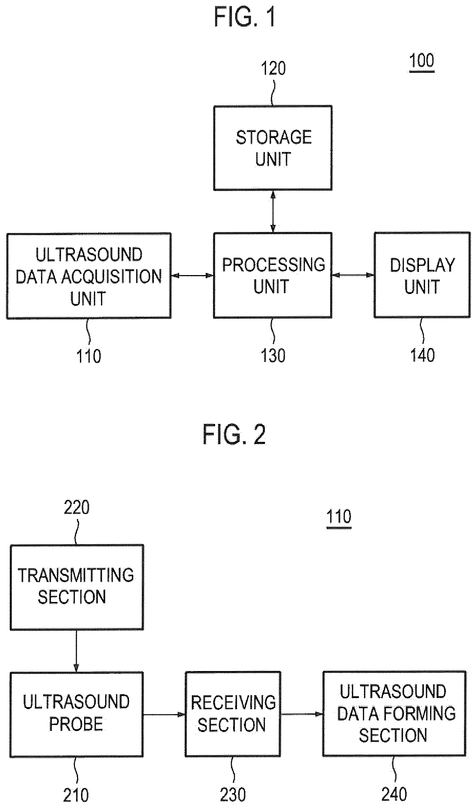

Referring to FIG. 1, an ultrasound system 100 in accordance with an illustrative embodiment is shown. As depicted therein, the ultrasound system 100 includes an ultrasound data acquisition unit 110.

The ultrasound data acquisition unit 110 is configured to transmit ultrasound signals to a living body. The living body includes target objects (e.g., a heart, a liver, blood flow, a blood vessel, etc.). The ultrasound data acquisition unit 110 is further configured to receive ultrasound signals (i.e., ultrasound echo signals) from the living body to acquire ultrasound data.

FIG. 2 is a block diagram showing an illustrative embodiment of the ultrasound data acquisition unit. Referring to FIG. 2, the ultrasound data acquisition unit 110 includes an ultrasound probe 210.

The ultrasound probe 210 includes a plurality of elements (not shown) for reciprocally converting between ultrasound signals and electrical signals. The ultrasound probe 210 is configured to transmit the ultrasound signals to the living body. The ultrasound probe 210 is further configured to receive the ultrasound echo signals from the living body to output electrical signals ("received signals"). The received signals are analog signals. The ultrasound probe 210 includes a three-dimensional mechanical probe or a two-dimensional array probe. However, it should be noted herein that the ultrasound probe 210 may not be limited thereto.

The ultrasound data acquisition unit 110 further includes a transmitting section 220. The transmitting section 220 is configured to control the transmission of the ultrasound signals. The transmitting section 220 is further configured to generate electrical signals ("transmitting signals") for obtaining an ultrasound image in consideration of the elements and focusing points. Thus, the ultrasound probe 210 converts the transmitting signals into the ultrasound signals, transmits the ultrasound signals to the living body and receives the ultrasound echo signals from the living body to output the received signals.

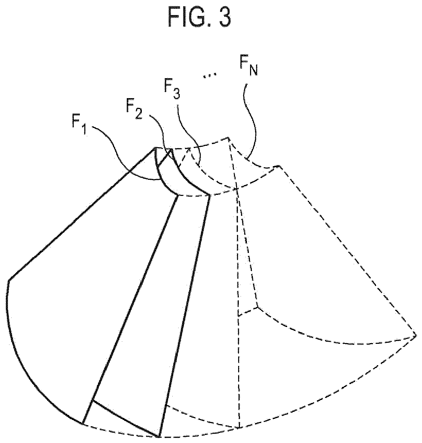

In one embodiment, the transmitting section 220 generates the transmitting signals for obtaining a plurality of frames $F_i$ ($1 \le i \le N$) corresponding to a three-dimensional ultrasound image at every predetermined time, as shown in FIG. 3. FIG. 3 is a schematic diagram showing an example of acquiring ultrasound data corresponding to the plurality of frames $F_i$ ($1 \le i \le N$). The plurality of frames $F_i$ ($1 \le i \le N$) represents sectional planes of the living body (not shown).

Referring back to FIG. 2, the ultrasound data acquisition unit 110 further includes a receiving section 230. The receiving section 230 is configured to convert the received signals provided from the ultrasound probe 210 into digital signals. The receiving section 230 is further configured to apply delays to the digital signals in consideration of the elements and the focusing points to output digital receive-focused signals.

The ultrasound data acquisition unit 110 further includes an ultrasound data forming section 240. The ultrasound data forming section 240 is configured to form ultrasound data based on the digital receive-focused signals provided from the receiving section 230. The ultrasound data include radio frequency data. However, it should be noted herein that the ultrasound data may not be limited thereto.

In one embodiment, the ultrasound data forming section 240 forms the ultrasound data corresponding to each of frames $F_i$ ($1 \le i \le N$) based on the digital receive-focused signals provided from the receiving section 230.

Referring back to FIG. 1, the ultrasound system 100 further includes a storage unit 120. The storage unit 120 stores the ultrasound data acquired by the ultrasound data acquisition unit 110. The storage unit 120 further stores a three-dimensional color reference table. The three-dimensional color reference table is a table for providing colors corresponding to three-dimensional coordinates of a three-dimensional coordinate system that includes an X-axis of depth, a Y-axis of an intensity accumulation value and a Z-axis of a shading value.

The ultrasound system 100 further includes a processing unit 130 in communication with the ultrasound data acquisition unit 110 and the storage unit 120. The processing unit 130 includes a central processing unit, a microprocessor, a graphic processing unit and the like.

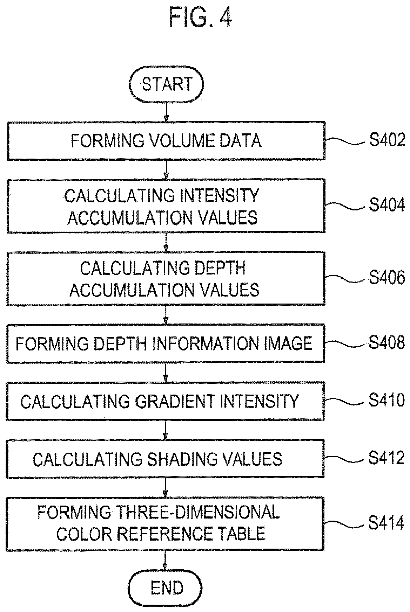

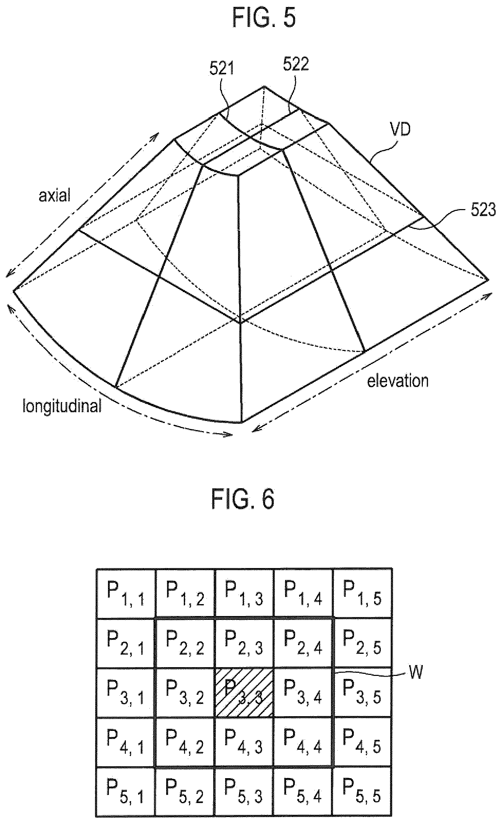

FIG. 4 is a flow chart showing a process of forming a three-dimensional color reference table. The processing unit 130 is configured to synthesize the ultrasound data corresponding to each of the frames $F_i$ ($1 \le i \le N$) to form volume data VD as shown in FIG. 5, at step S402 in FIG. 4.

FIG. 5 is a schematic diagram showing an example of the volume data. The volume data VD include a plurality of voxels (not shown) having brightness values. In FIG. 5, reference numerals 521, 522 and 523 represent an A plane, a B plane and a C plane, respectively. The A plane 521, the B plane 522 and the C plane 523 are mutually orthogonal. Also, in FIG. 5, the axial direction is a transmitting direction of the ultrasound signals, the lateral direction is a longitudinal direction of the elements, and the elevation direction is a swing direction of the elements, i.e., a depth direction of the three-dimensional ultrasound image.

Referring back to FIG. 4, the processing unit 130 is configured to perform volume-rendering upon the volume data VD to calculate intensity accumulation values throughout the depth, at step S404 in FIG. 4. Volume-rendering includes ray-casting for emitting virtual rays to the volume data VD.

In one embodiment, the processing unit 130 accumulates intensity values of sample points on each of the virtual rays based on transparency (or opacity) of the sample points to calculate the intensity accumulation values throughout the depth as equation 1 provided below.

$$\sum_{i}^{n} I_i \prod_{j}^{i-1} T_j \qquad\qquad (1)$$

In equation 1, I represents intensity, and T represents transparency.

The processing unit 130 is configured to perform ray-casting upon the volume data VD to calculate depth accumulation values throughout the depth, at step S406 in FIG. 4. The processing unit 130 is configured to form a depth information image based on the depth accumulation values, at step S408 in FIG. 4. The methods of forming the depth information image are well known in the art. Thus, they have not been described in detail so as not to unnecessarily obscure the present disclosure.

In one embodiment, the processing unit 130 accumulates depth values of the sample points on each of the virtual rays based on transparency (or opacity) of the sample points to calculate the depth accumulation values throughout the depth as equation 2 provided below.

$$\sum_{i}^{n} D_i \prod_{j}^{i-1} T_j \qquad\qquad (2)$$

In equation 2, D represents depth, and T represents transparency.

The processing unit 130 is configured to calculate gradient intensity based on the depth information image, at step S410 in FIG. 4. Generally, the depth information image is regarded as a surface having a height value corresponding to each of the pixels, and the gradient in the three-dimensional volume is regarded as a normal of the surface.

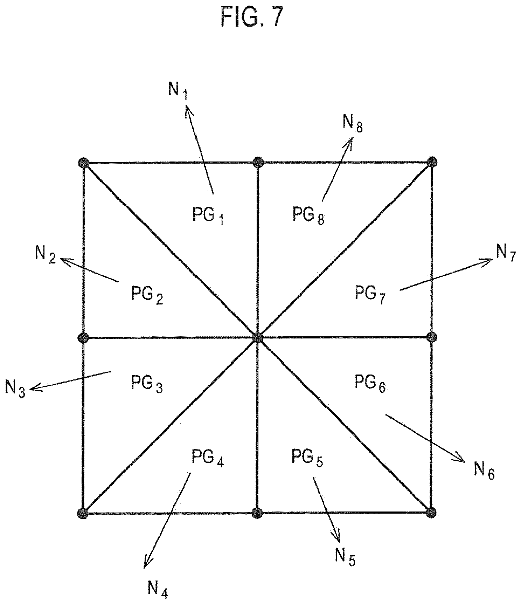

In one embodiment, the processing unit 130 sets a window W on the adjacent pixels $P_{2,2}$, $P_{2,3}$, $P_{2,4}$, $P_{3,2}$, $P_{3,4}$, $P_{4,2}$, $P_{4,3}$ and $P_{4,4}$ based on a pixel $P_{3,3}$ as shown in FIG. 6. The processing unit 130 further sets a center point corresponding to each of pixels within the window W as shown in FIG. 7. The processing unit 130 further sets polygons $PG_1$ to $PG_8$ for connecting the adjacent pixels based on the center points. The processing unit 130 further calculates normals $N_1$ to $N_8$ corresponding to the polygons $PG_1$ to $PG_8$. The methods of calculating the normal are well known in the art. Thus, they have not been described in detail so as not to unnecessarily obscure the present disclosure. The processing unit 130 further calculates a mean normal of the calculated normals $N_1$ to $N_8$. The processing unit 130 further sets the calculated mean normal as the surface normal (i.e., gradient intensity) of the pixel $P_{3,3}$.

Although it is described that the processing unit 130 sets 8 pixels as the adjacent pixels based on the each of the pixels, the number of the adjacent pixels may not be limited thereto. Also, although it is described that the polygons for connecting the adjacent pixels are a triangle, the polygons may not be limited thereto.

The processing unit 130 is configured to calculate shading values based on the surface normals and the virtual rays, at step S412 in FIG. 4. In one embodiment, the processing unit 130 calculates scalar product values between vectors of the surface normals and vectors of the virtual rays as the shading values.

The processing unit 130 is configured to form the three-dimensional color reference table based on the intensity accumulation values and the shading values, at step S414 in FIG. 4. In one embodiment, the processing unit 130 forms the three-dimensional color reference. The three-dimensional color reference table is stored in the storage unit 120.

Optionally, the processing unit 130 is configured to analyze the volume data VD to detect a skin tone of the target object (e.g., a fetus). The processing unit 130 is further configured to apply the detected skin tone to the three-dimensional color reference table. The methods of detecting the skin tone are well known in the art. Thus, they have not been described in detail so as not to unnecessarily obscure the present disclosure.

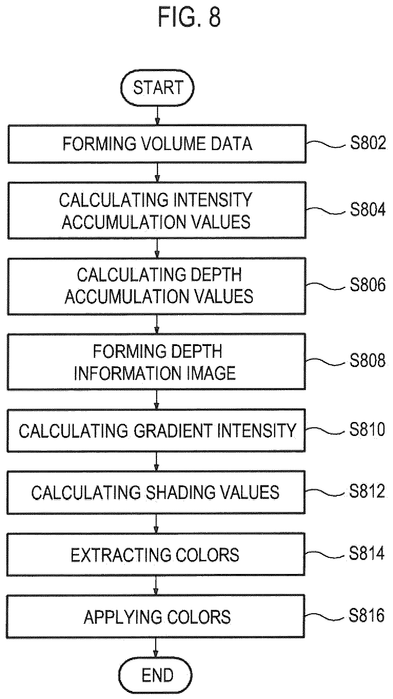

FIG. 8 is a flow chart showing a process of forming a three-dimensional ultrasound image. The processing unit 130 is configured to form the volume data VD as shown in FIG. 5 based on the ultrasound data newly provided from the ultrasound data acquisition unit 110, at step S802 in FIG. 8.

The processing unit 130 is configured to perform volume rendering (i.e., ray-casting) upon the volume data VD to calculate the intensity accumulation values throughout the depth, at step S804 in FIG. 8. The processing unit 130 is configured to perform ray-casting upon the volume data VD to form the depth accumulation values throughout the depth, at step S806 in FIG. 8. The processing unit 130 is configured to form the depth information image based on the depth accumulation values, at step S808 in FIG. 8. The processing unit 130 is configured to the gradient intensity based on the depth information image, at step S810 in FIG. 8. The processing unit 130 is configured to calculate the shading values based on the surface normals and the virtual rays, at step S812 in FIG. 8.

The processing unit 130 is configured to retrieve the three-dimensional color reference table stored in the storage unit 120 to extract colors corresponding to the intensity accumulation values and the shading values throughout the depth, at step S814 in FIG. 8.

Optionally, the processing unit 130 is configured to analyze the volume data VD to detect the skin tone of the target object (e.g., fetus). The processing unit 130 is further configured to retrieve the three-dimensional color reference table to extract colors corresponding to the skin tone.

Also, the processing unit 130 is configured to detect the skin tone (e.g., fetus) of the target object based on input information provided from a user input unit (not shown). The input information is skin tone selection information for selecting the skin tone of parents or a race.

The processing unit 130 is configured to apply the extracted colors to the volume data VD to form a three-dimensional ultrasound image, at step S816 in FIG. 8. In one embodiment, the processing unit 130 applies the extracted colors to the voxels corresponding to the depth of the volume data VD to form the three-dimensional ultrasound image.

Referring back to FIG. 1, the ultrasound system 100 further includes a display unit 140. The display unit 140 is configured to display the three-dimensional ultrasound image formed by the processing unit 130. The display unit 140 includes a cathode ray tube, a liquid crystal display, a light emitting diode, an organic light emitting diode and the like.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system (100), comprising:

   an ultrasound data acquisition unit (110) configured to transmit ultrasound signals to a target object via a probe (210), form ultrasound data corresponding to the target object based on ultrasound echo signals received from the target object;
   **Characterized in that** the system also comprises:

   a storage unit (120) for storing a three-dimensional color reference table for providing colors corresponding to at least one of intensity accumulation values and shading values throughout depth; and
   a processing unit (130) configured to form volume data based on the ultrasound data formed from the ultrasound data acquisition unit (110),
   **characterized in that** the processing unit (130) is configured to perform ray-casting for emitting virtual rays on the volume data, calculate intensity accumulation values by accumulating intensity values of sample points on each of the virtual rays, calculate depth accumulation values by accumulating depth values of the sample points on each of the virtual rays, form a depth information image based on the depth accumulation values, calculate surface normals corresponding to pixels of the depth information image, calculate scalar product values between vectors of the surface normals and vectors of the virtual rays as shading values, retrieve the three-dimensional color reference table to extract the colors corresponding to the intensity accumulation values and the shading values, and apply the extracted colors to the volume data to form a three-dimensional ultrasound image.

2. The ultrasound system (100) of Claim 1, wherein the processing unit (130) is configured to form the three-dimensional color reference table based on the volume data.

3. The ultrasound system (100) of Claim 2, wherein the processing unit (130) is configured to:

   perform the ray-casting for emitting virtual rays upon volume data to calculate the intensity accumulation values and the depth accumulation values throughout the depth;
   form the depth information image based on the depth accumulation values;
   calculate the surface normals based on the depth information image;
   calculate the shading values based on the surface normals; and
   form the three-dimensional color reference table based on the depth, the intensity accumulation values and the shading values.

**4.** The ultrasound system (100) of Claim 3, wherein the processing unit (130) is configured to:

set a window on adjacent pixels based on each of the pixels of the depth information image;
set a center point of each of the pixels within the windows;
set a plurality of polygons for connecting adjacent pixels based on the center points;
calculate a plurality of normals corresponding to the plurality of polygons;
calculate a mean normal of the normals;
set the mean normal as the surface normal corresponding to each of the pixels of the depth information image; and
calculate the scalar product values between vectors of the surface normals and vectors of the virtual rays as the shading value.

**5.** A method of providing a three-dimensional ultrasound image, comprising:

transmitting ultrasound signals to a target object via a probe;
forming ultrasound data corresponding to the target object based on ultrasound echo signals received from the target object;
forming volume data based on the ultrasound data corresponding to the target object;
performing ray-casting for emitting virtual rays on the volume data; **Characterized in that** the method also comprises:

calculating intensity accumulation values by accumulating intensity values of sample points on each of the virtual rays;
calculating depth accumulation values by accumulating depth values of the sample points on each of the virtual rays;
forming a depth information image based on the depth accumulation values;
calculating surface normals corresponding to pixels of the depth information image;
calculating scalar product values between vectors of the surface normals and vectors of the virtual rays as shading values;
retrieving a three-dimensional color reference table for providing colors corresponding to at least one of intensity accumulation values and shading values throughout depth to extract the colors corresponding to the intensity accumulation values and the shading values; and
applying the extracted colors to the volume data to form a three-dimensional ultrasound image.

**6.** The method of Claim 5, further comprising:
forming the three-dimensional color reference table based on the volume data, prior to performing the forming of the volume data.

**7.** The method of Claim 6, wherein the forming the three-dimensional color reference table comprises:

performing the ray-casting for emitting virtual rays upon volume data to calculate the intensity accumulation values and the depth accumulation values throughout the depth;
forming the depth information image based on the depth accumulation values;
calculating the surface normals based on the depth information image;
calculating the shading values based on the surface normals; and
forming the three-dimensional color reference table based on the depth, the intensity accumulation values and the shading values.

**8.** The method of Claim 7, wherein the calculating the surface normals comprises:

setting a window on adjacent pixels based on each of pixels of the depth information image;
setting a center point of each of the pixels within the windows;
setting a plurality of polygons for connecting adjacent pixels based on the center points;
calculating a plurality of normals corresponding to the plurality of polygons;
calculating a mean normal of the normals; and
setting the mean normal as the surface normal corresponding to each of the pixels of the depth information image.

**9.** The method of Claim 8, wherein the calculating the shading values comprises:
calculating scalar product values between vectors of the surface normals and vectors of the virtual rays as the shading value.

**Patentansprüche**

1. Ultraschallsystem (100), welches Folgendes aufweist:

eine Ultraschalldatenerlangungseinheit (110), die dafür vorgesehen ist, mittels einer Sonde (210) Ultraschallsignale zu einem Zielobjekt zu übertragen, Ultraschalldaten, die mit dem Zielobjekt korrespondieren, basierend auf von dem Zielobjekt empfangenen Ultraschallechosignalen zu erzeugen;
**dadurch gekennzeichnet, dass**
das System auch Folgendes aufweist:

eine Speichereinheit (120) zum Speichern einer dreidimensionalen Farbreferenztabelle zur Erzeugung von Farben, die mit wenigstens einem von Intensitätsansammlungswerten und Schattierungswerten durch die Tiefe hindurch korrespondieren; und
eine Verarbeitungseinheit (130), die dafür vorgesehen ist, Volumendaten basierend auf den Ultraschalldaten zu erzeugen, die durch die Ultraschalldatenerlangungseinheit (110) erzeugt wurden,
**dadurch gekennzeichnet, dass**
die Verarbeitungseinheit (130) dafür vorgesehen ist, Raycasting zum Aussenden virtueller Strahlen auf die Volumendaten durchzuführen, Intensitätsansammlungswerte durch Ansammeln von Intensitätswerten von Abtastpunkten auf jedem der virtuellen Strahlen zu berechnen, Tiefenansammlungswerte durch Ansammeln von Tiefenwerten der Abtastpunkte auf jedem der virtuellen Strahlen zu berechnen, ein Tiefeninformationsbild basierend auf den Tiefenansammlungswerten zu erzeugen, Flächennormalen zu berechnen, die mit Pixeln auf dem Tiefeninformationsbild korrespondieren, Skalarproduktwerte zwischen Vektoren der Flächennormalen und Vektoren der virtuellen Strahlen als Schattierungswerte zu berechnen, die dreidimensionale Farbreferenztabelle abzurufen, um die Farben zu extrahieren, die mit den Intensitätsansammlungswerten und den Schattierungswerten korrespondieren, und die extrahierten Farben an den Volumendaten anzuwenden, um ein dreidimensionales Ultraschallbild zu erzeugen.

2. Ultraschallsystem (100) nach Anspruch 1, wobei die Verarbeitungseinheit (130) dafür vorgesehen ist, die dreidimensionale Farbreferenzentabelle basierend auf den Volumendaten zu erzeugen.

3. Ultraschallsystem (100) nach Anspruch 2, wobei die Verarbeitungseinheit (130) für Folgendes vorgesehen ist:

Durchführen des Raycasting zum Ausgeben virtueller Strahlen auf die Volumendaten, um die Intensitätsansammlungswerte und die Tiefenansammlungswerte durch die Tiefe hindurch zu berechnen;
Erzeugen des Tiefeninformationsbilds basierend auf den Tiefenansammlungswerten;
Berechnen der Flächennormalen basierend auf dem Tiefeninformationsbild;
Berechnen der Schattierungswerte basierend auf den Flächennormalen;
Erzeugen der dreidimensionalen Farbreferenztabelle basierend auf der Tiefe, den Intensitätsansammlungswerten und den Schattierungswerten.

4. Ultraschallsystem (100) nach Anspruch 3, wobei die Verarbeitungseinheit (130) für Folgendes vorgesehen ist:

Festlegen eines Fensters auf benachbarten Pixeln basierend auf jedem der Pixel des Tiefeninformationsbilds;
Festlegen eines Mittelpunkts von jedem der Pixel innerhalb des Fensters;
Festlegen einer Vielzahl von Polygonen zum Verbinden benachbarter Pixel basierend auf den Mittelpunkten;
Berechnen einer Vielzahl von Normalen, die mit der Vielzahl von Polygonen korrespondieren;
Berechnen einer Durchschnittsnormalen der Normalen;
Festlegen der Durchschnittsnormalen als die Flächennormale, die mit jedem der Pixel des Tiefeninformationsbilds korrespondiert; und
Berechnen der Skalarproduktwerte zwischen Vektoren der Flächennormalen und Vektoren der virtuellen Strahlen als den Schattierungswerten.

5. Verfahren zum Erzeugen eines dreidimensionalen Ultraschallbilds, welches Folgendes aufweist:

Übertragen von Ultraschallsignalen zu einem Zielobjekt mittels einer Sonde;

Erzeugen von Ultraschalldaten, die mit dem Zielobjekt korrespondieren, basierend auf von dem Zielobjekt empfangenen Ultraschallechosignalen;

Erzeugen von Volumendaten basierend auf den Ultraschalldaten, die mit dem Zielobjekt korrespondieren;

Durchführen eines Raycasting zum Aussenden virtueller Strahlen auf die Volumendaten;

**dadurch gekennzeichnet, dass**

das Verfahren auch Folgendes aufweist:

Berechnen von Intensitätsansammlungswerten durch Ansammeln von Intensitätswerten von Abtastpunkten auf jedem der virtuellen Strahlen;

Berechnen von Tiefenansammlungswerten durch Ansammeln von Tiefenwerten der Abtastpunkte auf jedem der virtuellen Strahlen;

Erzeugen eines Tiefeninformationsbilds basierend auf den Tiefenansammlungswerten;

Berechnen von Flächennormalen, die mit Pixeln auf dem Tiefeninformationsbild korrespondieren;

Berechnen von Skalarproduktwerten zwischen Vektoren der Flächennormalen und Vektoren der virtuellen Strahlen als Schattierungswerte;

Abrufen einer dreidimensionalen Farbreferenztabelle zum Erzeugen von Farben, die mit wenigstens einem von Intensitätsansammlungswerten und Schattierungswerten durch die Tiefe hindurch korrespondieren, um die Farben zu extrahieren, die mit den Intensitätsansammlungswerten und den Schattierungswerten korrespondieren; und

Anwenden der extrahierten Farben an den Volumendaten, um ein dreidimensionales Ultraschallbild zu erzeugen.

6. Verfahren nach Anspruch 5, welches des Weiteren Folgendes aufweist:
Erzeugen der dreidimensionalen Farbreferenztabelle basierend auf den Volumendaten vor dem Durchführen des Erzeugens der Volumendaten.

7. Verfahren nach Anspruch 6, wobei das Erzeugen der dreidimensionalen Farbreferenztabelle Folgendes aufweist:

Durchführen des Raycasting zum Ausgeben virtueller Strahlen auf die Volumendaten, um die Intensitätsansammlungswerte und die Tiefenansammlungswerte durch die Tiefe hindurch zu berechnen;

Erzeugen des Tiefeninformationsbilds basierend auf den Tiefenansammlungswerten;

Berechnen der Flächennormalen basierend auf dem Tiefeninformationsbild;

Berechnen der Schattierungswerte basierend auf den Flächennormalen;

Erzeugen der dreidimensionalen Farbreferenztabelle basierend auf der Tiefe, den Intensitätsansammlungswerten und den Schattierungswerten.

8. Verfahren nach Anspruch 7, wobei das Berechnen der Oberflächennormalen Folgendes aufweist:

Festlegen eines Fensters auf benachbarten Pixeln basierend auf jedem der Pixel des Tiefeninformationsbilds;

Festlegen eines Mittelpunkts von jedem der Pixel innerhalb des Fensters;

Festlegen einer Vielzahl von Polygonen zum Verbinden benachbarter Pixel basierend auf den Mittelpunkten;

Berechnen einer Vielzahl von Normalen, die mit der Vielzahl von Polygonen korrespondieren;

Berechnen einer Durchschnittsnormalen der Normalen; und

Festlegen der Durchschnittsnormalen als die Flächennormale, die mit jedem der Pixel des Tiefeninformationsbilds korrespondiert.

9. Verfahren nach Anspruch 8, wobei das Berechnen der Schattierungswerte Folgendes aufweist:
Berechnen der Skalarproduktwerte zwischen Vektoren der Flächennormalen und Vektoren der virtuellen Strahlen als den Schattierungswerten.

**Revendications**

1. Système à ultrasons (100), comprenant :

une unité d'acquisition de données ultrasonores (110) configurée pour transmettre des signaux ultrasonores à un objet cible via une sonde (210), former des données ultrasonores correspondant à l'objet cible sur la base de signaux d'écho ultrasonores reçus de l'objet cible ;

**caractérisé en ce que** le système comprend également :

une unité de stockage (120) pour stocker une table de référence de couleurs tridimensionnelle pour fournir des couleurs correspondant à au moins une des valeurs d'accumulation d'intensité et des valeurs d'ombrage sur toute la profondeur ; et
une unité de traitement (130) configurée pour former des données de volume sur la base des données ultrasonores formées à partir de l'unité d'acquisition de données ultrasonores (110),
**caractérisé en ce que** l'unité de traitement (130) est configurée pour effectuer une diffusion de rayons pour l'émission de rayons virtuels sur les données de volume, calculer des valeurs d'accumulation d'intensité en accumulant des valeurs d'intensité de points d'échantillonnage sur chacun des rayons virtuels, calculer des valeurs d'accumulation de profondeur en accumulant des valeurs de profondeur des points d'échantillonnage sur chacun des rayons virtuels, former une image d'information de profondeur sur la base des valeurs d'accumulation de profondeur, calculer des normales de surface correspondant aux pixels de l'image d'information de profondeur, calculer les valeurs de produit scalaire entre les vecteurs des normales de surface et les vecteurs des rayons virtuels comme valeurs d'ombrage, récupérer la table de référence de couleurs tridimensionnelle pour extraire les couleurs correspondant aux valeurs d'accumulation d'intensité et aux valeurs d'ombrage, et appliquer les couleurs extraites aux données de volume pour former une image échographique tridimensionnelle.

2. Système à ultrasons (100) selon la revendication 1, dans lequel l'unité de traitement (130) est configurée pour former la table de référence de couleurs tridimensionnelle sur la base des données de volume.

3. Système à ultrasons (100) selon la revendication 2, dans lequel l'unité de traitement (130) est configurée pour :

effectuer la diffusion des rayons pour émettre des rayons virtuels sur des données de volume afin de calculer les valeurs d'accumulation d'intensité et les valeurs d'accumulation de profondeur sur toute la profondeur ;
former l'image d'information de profondeur sur la base des valeurs d'accumulation de profondeur ;
calculer les normales de surface sur la base de l'image d'information de profondeur ;
calculer les valeurs d'ombrage sur la base des normales de surface ; et former
la table de référence de couleurs tridimensionnelle sur la base de la profondeur, des valeurs d'accumulation d'intensité et des valeurs d'ombrage.

4. Système à ultrasons (100) selon la revendication 3, dans lequel l'unité de traitement (130) est configurée pour :

définir une fenêtre sur des pixels adjacents sur la base de chacun des pixels de l'image d'information de profondeur ;
définir un point central de chacun des pixels à l'intérieur des fenêtres ;
définir une pluralité de polygones pour relier des pixels adjacents sur la base des points centraux ;
calculer une pluralité de normales correspondant à la pluralité de polygones ;
calculer une normale moyenne des normales ;
définir la normale moyenne comme la normale de surface correspondant à chacun des pixels de l'image d'information de profondeur ; et
calculer les valeurs de produit scalaire entre les vecteurs des normales de surface et les vecteurs des rayons virtuels comme valeur d'ombrage.

5. Procédé pour fournir une image ultrasonore tridimensionnelle, comprenant :

la transmission de signaux ultrasonores à un objet cible via une sonde ;
la formation de données ultrasonores correspondant à l'objet cible sur la base de signaux d'écho ultrasonore reçus de l'objet cible ;
la formation de données de volume sur la base des données ultrasonores correspondant à l'objet cible ;
la diffusion de rayons pour l'émission de rayons virtuels sur les données de volume ; **caractérisé en ce que** le procédé comprend également :

le calcul des valeurs d'accumulation d'intensité en accumulant des valeurs d'intensité de points d'échantillonnage sur chacun des rayons virtuels ;
le calcul des valeurs d'accumulation de profondeur en accumulant des valeurs de profondeur des points d'échantillonnage sur chacun des rayons virtuels ;

la formation d'une image d'information de profondeur sur la base des valeurs d'accumulation de profondeur ;
le calcul des normales de surface correspondant aux pixels de l'image d'information de profondeur ;
le calcul des valeurs de produit scalaire entre les vecteurs des normales de surface et les vecteurs des rayons virtuels comme valeurs d'ombrage ;
la récupération d'une table de référence de couleurs tridimensionnelle pour fournir des couleurs correspondant à au moins l'une des valeurs d'accumulation d'intensité et des valeurs d'ombrage sur toute la profondeur pour extraire les couleurs correspondant aux valeurs d'accumulation d'intensité et aux valeurs d'ombrage ; et
l'application des couleurs extraites aux données de volume pour former une image ultrasonore tridimensionnelle.

6. Procédé selon la revendication 5, comprenant en outre :
la formation de la table de référence de couleurs tridimensionnelle sur la base des données de volume, avant d'effectuer la formation des données de volume.

7. Procédé selon la revendication 6, dans lequel la formation de la table de référence de couleurs tridimensionnelle comprend :

l'exécution de la diffusion des rayons pour émettre des rayons virtuels sur des données de volume afin de calculer les valeurs d'accumulation d'intensité et les valeurs d'accumulation de profondeur sur toute la profondeur ;
la formation de l'image d'information de profondeur sur la base des valeurs d'accumulation de profondeur ;
le calcul des normales de surface sur la base de l'image d'information de profondeur ;
le calcul des valeurs d'ombrage sur la base des normales de surface ; et la
formation de la table de référence de couleurs tridimensionnelle sur la base de la profondeur, des valeurs d'accumulation d'intensité et des valeurs d'ombrage.

8. Procédé selon la revendication 7, dans lequel le calcul des normales de surface comprend :

la définition d'une fenêtre sur des pixels adjacents sur la base de chacun des pixels de l'image d'information de profondeur ;
la définition d'un point central de chacun des pixels à l'intérieur des fenêtres ;
la définition d'une pluralité de polygones pour relier des pixels adjacents sur la base des points centraux ;
le calcul d'une pluralité de normales correspondant à la pluralité de polygones ;
le calcul d'une normale moyenne des normales ; et
la définition de la normale moyenne comme la normale de surface correspondant à chacun des pixels de l'image d'information de profondeur.

9. Procédé selon la revendication 8, dans lequel le calcul des valeurs d'ombrage comprend :
le calcul des valeurs de produit scalaire entre les vecteurs des normales de surface et les vecteurs des rayons virtuels comme valeur d'ombrage.

# FIG. 1

120

100

STORAGE
UNIT

ULTRASOUND
DATA ACQUISITION
UNIT

PROCESSING
UNIT

DISPLAY
UNIT

110

130

140

# FIG. 2

220

110

TRANSMITTING
SECTION

ULTRASOUND
PROBE

RECEIVING
SECTION

ULTRASOUND
DATA FORMING
SECTION

210

230

240

FIG. 3

# FIG. 4

START

FORMING VOLUME DATA — S402

CALCULATING INTENSITY
ACCUMULATION VALUES — S404

CALCULATING DEPTH
ACCUMULATION VALUES — S406

FORMING DEPTH INFORMATION IMAGE — S408

CALCULATING GRADIENT INTENSITY — S410

CALCULATING SHADING VALUES — S412

FORMING THREE-DIMENSIONAL
COLOR REFERENCE TABLE — S414

END

## FIG. 5

## FIG. 6

FIG. 7

# FIG. 8

```
        START
          │
          ▼
┌─────────────────────────┐
│  FORMING VOLUME DATA    │────  S802
└─────────────────────────┘
          │
          ▼
┌─────────────────────────┐
│  CALCULATING INTENSITY  │────  S804
│  ACCUMULATION VALUES    │
└─────────────────────────┘
          │
          ▼
┌─────────────────────────┐
│  CALCULATING DEPTH      │────  S806
│  ACCUMULATION VALUES    │
└─────────────────────────┘
          │
          ▼
┌─────────────────────────┐
│  FORMING DEPTH          │────  S808
│  INFORMATION IMAGE      │
└─────────────────────────┘
          │
          ▼
┌─────────────────────────┐
│ CALCULATING GRADIENT INTENSITY │────  S810
└─────────────────────────┘
          │
          ▼
┌─────────────────────────┐
│ CALCULATING SHADING VALUES │────  S812
└─────────────────────────┘
          │
          ▼
┌─────────────────────────┐
│  EXTRACTING COLORS      │────  S814
└─────────────────────────┘
          │
          ▼
┌─────────────────────────┐
│  APPLYING COLORS        │────  S816
└─────────────────────────┘
          │
          ▼
         END
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2002172409 A1
- US 6559843 B1